(19) Europäisches Patentamt
European Patent Office
Office européen des brevets

(11) **EP 4 437 927 A1**

(12) **EUROPEAN PATENT APPLICATION**

(43) Date of publication:
**02.10.2024 Bulletin 2024/40**

(21) Application number: **23166161.2**

(22) Date of filing: **31.03.2023**

(51) International Patent Classification (IPC):
**A61B 1/00** (2006.01)    **A61B 1/04** (2006.01)
**A61B 1/045** (2006.01)    **A61B 1/06** (2006.01)

(52) Cooperative Patent Classification (CPC):
**A61B 1/000095; A61B 1/043; A61B 1/045;**
**A61B 1/0638; A61B 1/0655**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Erbe Vision GmbH**
**78573 Wurmlingen (DE)**

(72) Inventors:
• **Shirish Joshi**
**78573 Wurmlingen (DE)**
• **Faisal Kalim**
**72764 Reutlingen (DE)**

(74) Representative: **Rüger Abel Patentanwälte**
**PartGmbB**
**Webergasse 3**
**73728 Esslingen a. N. (DE)**

(54) **DEVICE AND METHOD FOR MEDICAL IMAGING**

(57)    The invention relates to a method and a device (1) for medical imaging in particular intra-operative imaging of tissue (21) of a patient's body. The device (1) comprises the illumination unit (2), the imaging unit (3), the processing unit (4) and the display unit (8). The imaging unit captures fluorescent images (I_fluo) and live images (I_live) of the same scenery. In the processing unit (4), the fluorescent images (I_fluo) are processed by using the detected motion between the tissue (21) and the imaging unit (3) based on the detected motion. The filtered fluorescent images (I_filt) are non-linearly mapped into the live images (I_live) in order to improve the visibility of fluorophores in the tissue resulting in an overlay image (I_over) that comprises details of the live images (I_live) on the one hand and details of the fluorescent images (I_fluo) on the other hand.

Fig. 1

EP 4 437 927 A1

**Description**

The present invention relates generally to methods, devices, systems and apparatuses for medical imaging, in particular intra-operative imaging of tissues of a patient's body.

Intra-operative medical imaging systems, such as endoscopic or laparoscopic imaging systems, typically comprise camera systems adapted to be placed close to or within a patient's body for capturing live images during the medical procedure or the surgery. Typically, the live images are captured within a visible light range such as having a wavelength in the range of 400-780 nanometers. In the tissue of the patient's body, there may be fluorescent features present that emit fluorescent light if excited by excitation light. The fluorescent light emitted by the fluorescent features may be captured in the live images, however, the fluorescent light is usually weak in the live images. Accordingly, intra-operative medical imaging systems typically comprise fluorescence camera sensors for capturing fluorescence images in the wavelength range at least a major part of the light emitted by the fluorescent features is expected to be, such that the fluorescent features are more clearly visible. The wavelength of the fluorescent light may be situated at least partly in the visible range and at least partly in the infrared light range such as in a wavelength range of 780 nm to 1000 nm.

US patent US 10 694 152 B2 describes an endoscopic video system with a camera comprising a single color image sensor for fluorescence and color imaging. The tissue under observation is illuminated continuously with fluorescence excitation light as well as periodically with visible light comprising wavelengths outside the fluorescence excitation wavelength range. The fluorescence and color images are displayed simultaneously which can be inconvenient for the person performing the operation, who must alternate between the fluorescence and the color images in order to gather all details in both images.

US patent application US 2019/167083 A1 describes an endoscopic system which includes a light source apparatus configured to emit excitation light for exciting a fluorescent medical agent and illumination light. The endoscopic system includes an image sensor configured to capture an image of fluorescent light emitted in response to the excitation light from the fluorescent medical agent and an image of the reflected light. A combined image is generated by mixing a first image of the reflected light with a second image of the fluorescent light in a set of non-overlapping pixel arrays.

Further endoscopic camera systems can be found, for instance, in JP 2012-085917 A, JP 2012-085916 A and JP 2003-070721 A.

In medical imaging, such as intra-operative imaging, the signals of the fluorescence images are usually weak and noisy compared to live images. Due to the comparatively low signal intensity and high noise intensity, it is possible that the surgical staff may miss important details in the fluorescence images because the visibility of the details may be affected by the noise.

US 2008/204 600 A1 describes an adaptive temporal noise reduction method that adaptively combines motion adaptive filtering results and motion compensated results in order to reduce Gaussian additive noise in video sequences.

US 2017/281 093 A1 further describes a method for de-noising an image stream of a moving structure in a medical device. In the method, the movement of the structure is measured by comparing two images each representing the same portion of the moved structure.

Moreover, there are approaches that reduce noise in images by frame addition. In this approach, a plurality of successive frames are added together to form a filtered image that includes a reduced level of noise. This approach can be found in JP 2012-085917 A, JP 2012-085916 A and EP 3469420 A1, US 2021/052226 A1. In JP 2012-085917 A, the number of the added frames depends on the motion detected.

Further prior art documents can be found in EP 3,834,407 A1, US 5,667,474 A, US 2019/167083 A1, JP H-07250804 A and JP 2003-070721 A.

However, generating overlay images from fluorescence live images and live images with a good visibility remains a challenge. Usually, the boundaries of fluorescence features are not clearly visible so that there is an increased risk that the person operating the imaging device will miss features, in particular of thin, delicate features, in the overlay image. On the other hand, strong fluorescence signals can result in a saturation of the overlay images so that details from the live images may no longer be visible due to strong fluorescence features.

Therefore, it is an objective of the present invention to provide an improved medical imaging apparatus and method in which the visualization of fluorescent images in an overlay image is enhanced.

The objective of the present invention is solved by the device for medical imaging according to claim 1 and the method for medical imaging according to claim 15:

The inventive device for medical imaging of tissue of a patient's body, in particular for intra-operative imaging, comprises an illumination unit, an imaging unit, a processing unit and a display unit.

The illumination unit is configured to illuminate an area of the tissue by emitting illumination light that includes at least excitation light that excites fluorescent features in the tissue to emit fluorescent light. The illumination light may be in the visible range of light, whereas the excitation light may be in the range of visible light or also in the range of invisible light, such as ultraviolet and infrared light. The excitation light may - for instance - comprise wavelengths that are larger than 500 nanometers or larger than 600 nanometers. For example, the wavelength of the excitation light may range from 600 to 800 nanometers, preferably 600 to 900 nanometers or 650 to 1350 nanometers, which means in the near infrared range. The excitation light may - for example - also comprise wavelengths that are at least 1400 nanometers, at least 3000 nanometers, at least 8000 nanometers or at least 15000 nanometers. The excitation light may be pulsed or continuous.

The fluorescent features in the tissue may - for instance - be fluorophores which are present in the tissue. For instance, the hemoglobin in blood vessels may be excited to fluoresce by the excitation light. The fluorescent features may also result from fluorescent dyes injected into the tissue or marker devices inserted into the patient's body.

The imaging unit is configured to capture fluorescent images and corresponding live images of the illuminated area. In particular, the imaging unit may comprise an image separation means configured to separate the fluorescent images from the live images such that the imaging unit is configured to capture fluorescent images and separate live images of the illuminated area at least substantially at the same time. For example, the image separation means can be a dichroic prism that splits the light which enters the imaging unit into a live image which - for instance - contains the images in the visible light range and fluorescent images, which - for instance - contain light in a fluorescent light range. The imaging unit may comprise separate sensors for capturing the fluorescent images and the images.

The processing unit is configured to receive the fluorescent images and the live images from the imaging unit. The processing unit further includes a motion detection module, an adaptive motion filter module and a mapping module. The motion detection module, the adaptive motion filter module and the mapping module may be implemented as software and/or hardware modules.

The motion detection module is configured to determine motion between the tissue and the imaging unit. The adaptive motion filter module is configured to generate filtered fluorescent images by applying motion based adaptive filtering to the fluorescent images. The mapping module is configured to generate overlay images by non-linearly mapping the filtered fluorescent images into the live images.

For determining the motion between the tissue and the imaging unit, the motion detection module, may evaluate changes in the image data of the imaging unit, for instance by applying and evaluating the optical flow or comparing particularly significant image features in-between frames. Additionally or alternatively, the motion detection module may receive and evaluate motion data from a motion sensor, such as an inertial measurement unit (IMU), which can be located in the camera head.

Motion between the tissue and the imaging unit may be present due to movement of the imaging unit relative to the tissue, movement of the tissue relative to the imaging unit, or both combined.

Moreover, the display unit is configured to receive and display the overlay images. The display unit may also be configured to additionally receive and display the fluorescent images and the live images.

An aspect of the present invention is based on the notion that the processing unit is configured to adaptively filter the fluorescent images by considering the motion between the tissue and the imaging unit. In the motion-based adaptive filtering, the processing unit may add previous and present fluorescent images, wherein at least one parameter for the addition of the fluorescent images depends on the motion determined by the motion detection module. The at least one parameter defines the influence of the present fluorescent image in relation to the previous filtered fluorescent image. In particular, if a comparably high motion value is determined, the at least one parameter is increased such that the present fluorescent image has a higher influence on the resulting filtered fluorescent image than the previous filtered fluorescent images. In contrast, if the determined motion value is relatively low, the at least one parameter is decreased such that the influence of the previous filtered fluorescent images is higher than the influence of the present fluorescent image in the resulting filtered fluorescent image.

Another aspect of the present invention is based on the notion that the processing unit is further configured to generate an overlay image in which the filtered fluorescent images are mapped into the live images in the mapping module, wherein the mapping is non-linear. With the non-linear mapping, it is possible to amplify regions in the filtered fluorescent images with lower intensity values more than regions with comparably higher intensity values. This may result in an amplification of the details in the overlay images without the occurrence of over-saturation. Accordingly, combining the motion-based filtering with the non-linear mapping results in an amplification of the fluorescent details in the overlay images, while the noise present in the fluorescent images can be effectively suppressed. The mapping module may also restrict the output values in the non-linear mapping of the filtered fluorescent images to a maximal value so that details from the live images may remain visible even when the filtered fluorescent images have a comparatively large intensity value.

Fluorescent images can be images that collect only light in a predetermined preferably narrow band wavelength range. The wavelength collected in the fluorescent images may be in the visible light range and/or the invisible light range. If the fluorescent images are in the visible light range, they may - for example - comprise wavelength larger than 500 nanometers. If the fluorescent images are in the infrared light range, they may - for example - comprise wavelengths that are larger than 600 nanometers, e.g. ranging from 600 to 800 nanometers, 600 to 900 nanometers or 650 to 1350 nanometers (i.e. the near infrared (NIR) range). The infrared light may - for example - also comprise wavelengths that are at least 1400 nanometers, at least 3000 nanometers, at least 8000 nanometers or at least 15000 nanometers.

In particular, the generation of the filtered fluorescent images in the adaptive motion filter module involves a weighted averaging operation of a present fluorescent image and a previous filtered fluorescent image, wherein the weights in the averaging are adapted to the motion determined. This allows effective suppression of noise in the fluorescent images without filtering out the details of the fluorescent features. Furthermore, trailing effects due to motion in the fluorescent images can be avoided, since the weights in the average operation are adjusted to the motion.

Preferably, the weights in the weighted averaging operation comprise a first factor and a second factor, wherein the first factor determines the influence of the present fluorescent image in relation to the previous filtered fluorescent image and the second factor determines the amplification of the previous filtered fluorescent image.

In particular, the adaptive motion filter module is configured to successively generate a filtered fluorescent image for a plurality of time steps, wherein the filtered fluorescent image is calculated for a present time step as follows:

$$\texttt{I\_filt} = \alpha \cdot \texttt{I\_fluo\_pres} + (1 - \alpha) \cdot \beta \cdot \texttt{I\_filt\_prev},$$

wherein I_filt is the filtered fluorescent image, I_fluo_pres is the present fluorescent image, I_filt_prev is the previous filtered image, $\alpha$ is the first factor and $\beta$ is the second factor. The filtered fluorescent image in the present time step preferably becomes the previous fluorescent filtered image in a next time step.

Preferably, the adaptive motion filter module is configured to adapt the first factor to the determined motion such that the first factor decreases when the motion decreases and the first factor increases when the motion increases. Accordingly, the first factor is proportional to the motion determined in the motion detection module.

In particular, the second factor is proportional to at least the first factor, preferably the second factor is also proportional to the previous filtered fluorescent image. For example, the second factor can be proportional to the brightness of the previous filtered fluorescent image. The lower the brightness of the previous filtered fluorescent image, the higher the value of the second factor. This leads to an enhancement of the brightness of the filtered image even in situations when the brightness of the present fluorescent image is rather low.

Preferably, the second factor is defined to be in a predetermined range, the predetermined range in particular being between 1.00 and 1.50 preferably between 1.00 and 1.30. Defining the second factor in a predetermined range leads to a stable amplification avoiding over-saturation of the fluorescent image signal. Accordingly, the second factor can be dynamically changed with the brightness of the filtered fluorescent images and the motion. Ensuring that the second factor is always above 1.00 can result in the adaptive motion filter module not attenuating the previous filtered fluorescent image signal.

In particular, the processing unit further comprises a pre-processing noise filter module configured to:

receive the fluorescent images from the imaging unit;

generate pre-filtered fluorescent images by applying a noise filter to the fluorescent images; and

transmit the pre-filtered fluorescent images to the adaptive motion filter module. The noise filter may - for instance - be a spatial median or Gaussian noise filter. So the pre-filtered fluorescent images are used instead of unfiltered fluorescent images. This can be a simple but yet effective way to further enhance the suppression of noise in the fluorescent images.

Furthermore, the mapping module is preferably configured to apply a non-linear amplification function on the filtered fluorescent images in the mapping of the filtered fluorescent images into the live images. In doing so, the mapping module can generate an overlay image in which lower intensity values of the filtered fluorescent image can be displayed with a stronger value. This particularly amplifies the lower signal values while avoiding oversaturation of relatively high signal values.

Preferably, the non-linear amplification function is under-linear homogenous. This means that the homogenous degree is below 1.00. The under-linear homogenous amplification function may result in lower intensity values being mapped with a higher value while higher intensity values are mapped with a lower value, in particular when regarding the interval between 0.00 and 1.00 of the amplification function. For instance, the non-linear amplification function may be a square root function or a cubic root function, or a root function of a higher degree.

Additionally or alternatively, the amplification function can be a non-linear function that is specifically adapted to amplify lower intensity values and attenuate higher intensity values compared to a linear mapping.

Preferably, the value range of the non-linear amplification function is limited to a predetermined maximal value. The predetermined maximal value particularly has a value below 1.00, preferably below 0.80, 0.70 or 0.50, so that the live image is displayed in the background of the overlay image even when the intensity value of the filtered fluorescent image is close to the maximum value. This ensures that details in the live images remain visible in the overlay images, even when the fluorescence signal is comparably large.

In particular, the mapping module is configured to assign the intensity values of the mapped and filtered fluorescent images to different visually distinguishable colors. The colors should be distinguishable to the naked eye. This can further improve the visualization of the resulting color map for the operating person. For example, the distinguishable colors may be multiple of the following: blue, green, orange, yellow, red, violet and brown.

The overlay images are preferably calculated for every time step as follows:

$$I\_over = (1 - \gamma(I\_filt)) \cdot I\_live + \gamma(I\_filt) \cdot color(I\_filt),$$

wherein $\gamma$ is the amplification function, color is the color assigning operator, I_filt is the filtered fluorescent image, I_live is the corresponding live image and I_over is the overlay image. The color assigning operator can be a linear mapping operator.

In a preferred embodiment, the device further comprises an endoscope with a distal end and a proximal end between which an elongated shaft extends. An inertial measurement unit may be positioned at the distal end of the endoscope or the laparoscope. The illumination unit and/or the imaging unit can be positioned at the distal end and/or the proximal end of the endoscope. In case the illumination unit and the imaging unit are located at the distal end of the endoscope, the endoscope comprises signal lines in the elongated shaft for connecting at least the imaging unit with the processing unit. Moreover, the endoscope may comprise signal lines in the elongated shaft of the endoscope for controlling the illumination unit. In case the illumination unit and the imaging unit are located at the proximal end of the endoscope, the endoscope comprises optical channels and/or optical objectives for passing the illumination light which contains the excitation light from the proximal end to the distal end of the endoscope as well as passing the reflected and/or fluorescence light back entering the optical objective at the distal end to the proximal end of the endoscope.

The inventive method for medical imaging of tissue of a patient's body, in particular for intra-operative imaging, preferably with the above-described device, includes the method steps of illuminating an area of the tissue with an illumination unit by emitting illumination light containing at least excitation light which excites fluorescent features, such as fluorophores, in the tissue to emit fluorescent light, capturing fluorescent images and (separate) corresponding live images of the illuminated area with an imaging unit as well as processing the fluorescent images and the live images including the following steps:

Determining the motion between the tissue and the imaging unit;

Generating filtered fluorescent images by applying motion-based adaptive filtering to the fluorescent images; and

Generating overlay images by non-linearly mapping the filtered fluorescent images into the live images; and

Displaying the generated overlay images.

All the features and advantages described with respect to the inventive device are equally applicable to the inventive method.

Further details and advantages can be taken from the drawings, the respective description and from the claims as well.

Examples of the embodiments of the invention are illustrated in the drawings in which

figure 1 illustrates a first example of the device for medical imaging of tissue of a patient's body

figure 2 illustrates a second example of the device for medical imaging of tissue of a patient's body

figure 3 illustrates an example for the fluorescent images, the live images, the filtered images and the overlay images

figure 4 illustrates examples of the amplification function

figure 5 illustrates an example for the information flow in the processing unit and

figure 6 illustrates an example of the imaging unit.

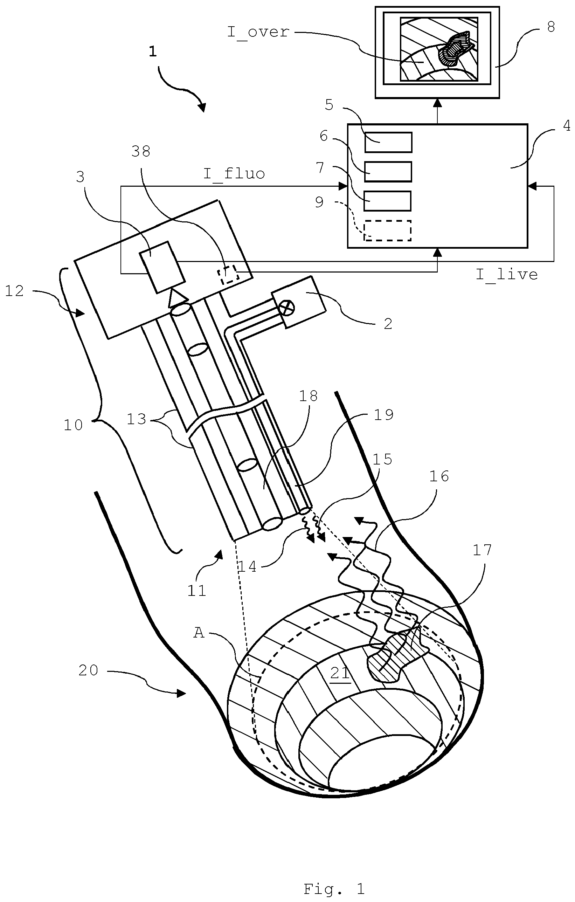

Figure 1 illustrates a first example of the inventive device 1 for medical imaging of tissue 21 of a patient's body. The device 1 comprises an illumination unit 2, an imaging unit 3, a processing unit 4 and a display unit 8.

In the displayed example of figure 1, the device 1 comprises an endoscope 10 such that the device 1 is configured for imaging the tissue 21 of a hollow vessel or organ 20 inside a patient's body.

The illumination unit 2 is configured to illuminate an area A of the tissue 21 by emitting illumination light 14 containing at least excitation light 15 which excites fluorophores in the tissue 21 to emit fluorescent light 16. In figure 1, a fluorescent feature 17 is present in the illuminated area A.

The imaging unit 3 is configured to capture fluorescent images I_fluo and corresponding live images I_live of the illuminated area A. The imaging unit 3 is connected with the processing unit 4 so that the processing unit 4 is configured to receive the fluorescent images I_fluo and the live images I_live from the imaging unit 3.

The processing unit 4 is configured to process the fluorescent images I_fluo and the live images I_live in order to form overlay images I_over. The processing unit 4 and the display unit 8 are connected such that the display unit is configured to receive the overlay images I_over.

The display unit 8 also comprises a screen for at least displaying the overlay images. The display unit 8 can also be configured to receive the live images I_live, the fluorescent images I_fluo and/or the filtered images I_filt. Additionally, the display unit 8 can be configured to display the live images I_live, the fluorescent images I_fluo and/or the filtered fluorescent images I_filt.

The processing unit 4 comprises a motion detection module 5, an adaptive motion filter module 6 and a mapping module 7. Optionally, the processing unit 4 may also comprise a pre-processing noise filter module 9.

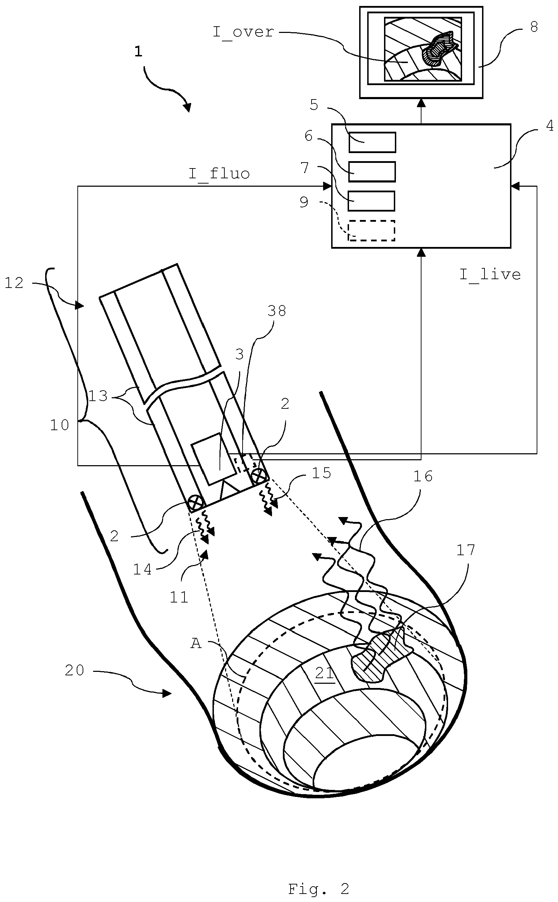

The motion detection module 5 is configured to determine motion between the tissue 21 and the imaging unit 3. The motion detection module 5 can be configured to determine motion in the fluorescent images I_fluo and/or the live images I_live, for example with image processing methods, such as the optical flow operator feature detection methods and/or tracking methods can be utilized. Alternatively, the motion detection module 5 may be configured to determine the motion by using an external tracking system, a motion sensor or an integrated inertial measurement unit (IMU). In the illustrated example, however, the motion detection module 5 is configured to detect the motion in the images by image processing. Also, the alternative example with an IMU 38 is illustrated in figures 1 and 2 with a dashed line.

The adaptive motion filter module 6 is configured to generate filtered fluorescent images I_filt by applying motion based adaptive filtering to the fluorescent images I_fluo. In the adaptive motion filter module 6, the fluorescent images I_fluo are continuously averaged. After initializing the filtered fluorescent images I_filt with an empty invention which contains only zeroes, the adaptive motion filter module forms a weighted sum of a present fluorescent image I_fluo_pres and a previous filtered image I_filt_prev, wherein the weights in the averaging are adapted to the motion determined in the motion detection module 5.

The mapping module 7 is configured to generate overlay images I_over by non-linearly mapping the filtered fluorescent images I_filt into the live images I_live.

The adaptive motion filter module 6 and the mapping module 7 are discussed in more detail below.

The device 1 displayed in figure 1 comprises an endoscope 10 with a distal end 11 and a proximal end 12, between which an elongated shaft 13 extends.

The illumination unit 2 and the imaging unit 3 are both positioned at the proximal end 12 of the endoscope 10 in figure 1. Accordingly, the illumination light 14 which is generated by an illumination light source in the illumination unit 2 is guided through an optical channel 19 from the proximal end 12 to the distal end 11. For the optical channel 19, an optical fiber may for instance be used. Moreover, the endoscope 10 includes an optical objective 18 through which the light reflected from the patient's tissue 21 and fluorescent light excited and emitted from fluorophores in the tissue 21 are guided from the distal end 11 of the endoscope 10 to the proximal end 12 of the endoscope 10, where the imaging unit 3 is positioned.

Figure 2 illustrates a second example of the device 1 for medical imaging. The second example differs from the first example in that the illumination source 2 and the imaging unit 3 are both located at the distal end 11 of the endoscope 10. For the second example, the same applies as described in relation to the first example using the reference signs.

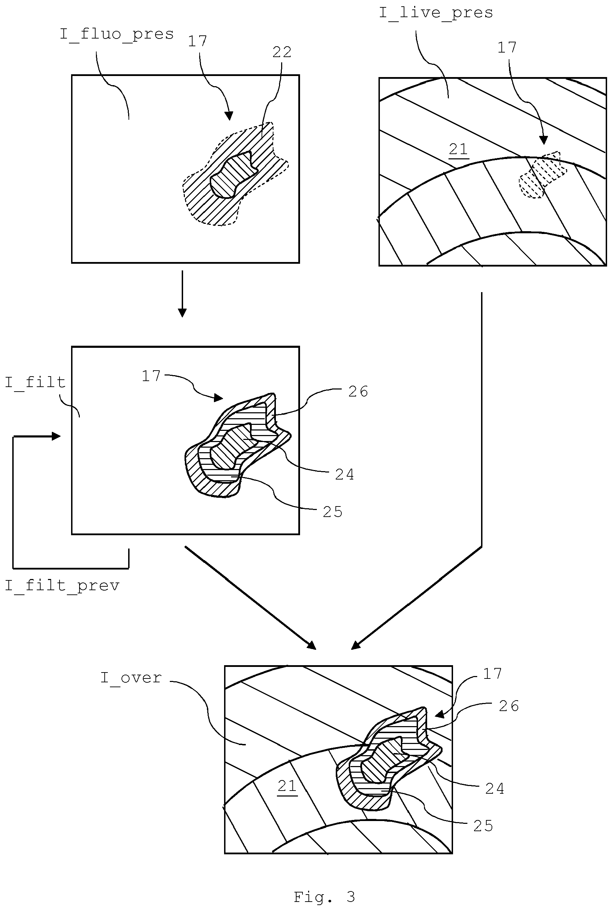

Figure 3 shows an example of the information flow on which the inventive idea is based. On the upper left side, a present fluorescent image I_fluo_pres is depicted. The present fluorescent image I_fluo_pres shows a fluorescent feature 17, wherein the border region 22 of the fluorescent feature 17 has a low intensity.

On the upper right side in figure 3, an example for a present live image I_live_pres is depicted. In the present live image I_live_pres, the tissue 21 of the patient's body can be seen clearly. However, the fluorescent feature 17 is difficult to see in the present live image I_live_pres.

On the middle left side of figure 3, an example of a filtered fluorescent image I_filt corresponding to the present fluorescent image I_fluo_pres is depicted. The filtered fluorescent image I_filt is calculated by continuously adding the fluorescent images I_fluo with a first factor $\alpha$ and which determines the influence of the present fluorescent image I_fluo_pres in relation to the previous filtered image I_filt_prev. The second factor $\beta$ determines the amplification of the previous filtered image I_filt_prev. Correspondingly, the filtered fluorescent image I_filt is calculated as follows:

$$I\_filt = \alpha \cdot I\_fluo\_pres + (1 - \alpha) \cdot \beta \cdot I\_filt\_prev,$$

wherein I_filt is the filtered fluorescent image, I_fluo_pres is the present fluorescent image, I_filt_prev is the previous filtered image, $\alpha$ is the first factor and $\beta$ is the second factor. The filtered fluorescent image I_filt of the present time step becomes the previous filtered image I_filt_previn the next time step.

In the mapping module 7, a non-linear amplification function $\gamma$ is applied on the filtered fluorescent image I_filt such that pixel with a lower intensive value are amplified more than pixels with a high intensity value. This may result in an amplification of the border regions 22 while an over amplification and therefore an over-saturation of the pixels with high intensity values can be avoided. In the mapping module 7, the intensity values of the mapped and filtered fluorescent images I_filt are assigned to different visually distinguishable color groups. As shown in figure 3, the border region 22 is split into three regions. The first color region 24 is of the highest intensity, wherein the second color 25 is assigned to intensity values of a middle range and the third color 26 is assigned to intensity values within a lower range.

On the lower end of figure 3, an example of the resulting overlay image I_over is depicted. The overlay image I_over shows the tissue 21 of the patient's body which comes from the present live image I_live_pres and also the fluorescent feature 17 which comes from the present fluorescent image I_fluo_pres. The fluorescent feature 17 is shown as a heat map comprising different distinguishable color groups that are assigned to the corresponding pixels within an intensity value range. In particular, the border region 22 can now be clearly seen in the overlay image I_over.

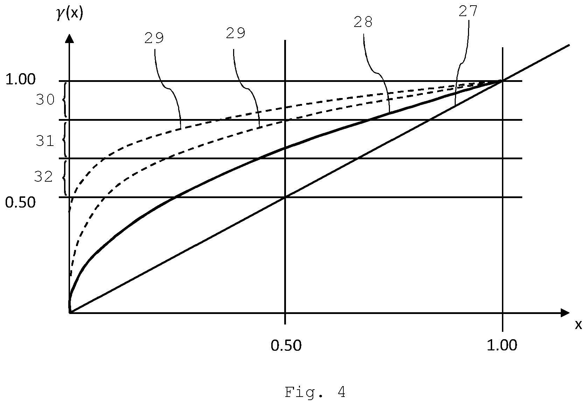

Figure 4 shows multiple examples for the non-linear amplification function $\gamma$ over the input x. As a reference in figure 4, the linear function 27 is shown. The amplification function $\gamma$ can be a square root function 28 for example. Alternatively, root functions of higher degrees 29 can be used as a amplification function $\gamma$. In figure 4, the square root function 28 is used so that low values as input values, such as a value of 0.35, result in higher values, such as 0.59. Higher values, such as 0.75, also result in a higher value, such as 0.87, however, the amplification of the higher value is less than the amplification of the lower values.

Moreover, figure 4 shows a first assigned range 30, a second assigned range 31 and a third assigned range 32. The color regions in the overlay images I_over are associated with the ranges in the amplification function $\gamma$. In this example, the first color region 24 in figure 3 is associated with the first range 30 in figure 4, the second color region 25 in figure 3 is associated with the second range 31 in figure 4 and the third color region 26 in figure 3 is associated with the third range 32 in figure 4. Pixels having an intensity value within the first assigned range 31 may for instance be displayed in brown in the overlay images. Pixels having an intensity value within the second assigned range 31 may be displayed in red in the overlay images. Pixels having an intensity value within the third assigned range 32 may be displayed in blue in the overlay images. Accordingly, the overlay images partly containing a heat map of the fluorescent features overlaid in the corresponding live images. This improves the visibility of fluorescent features in the overlay images.

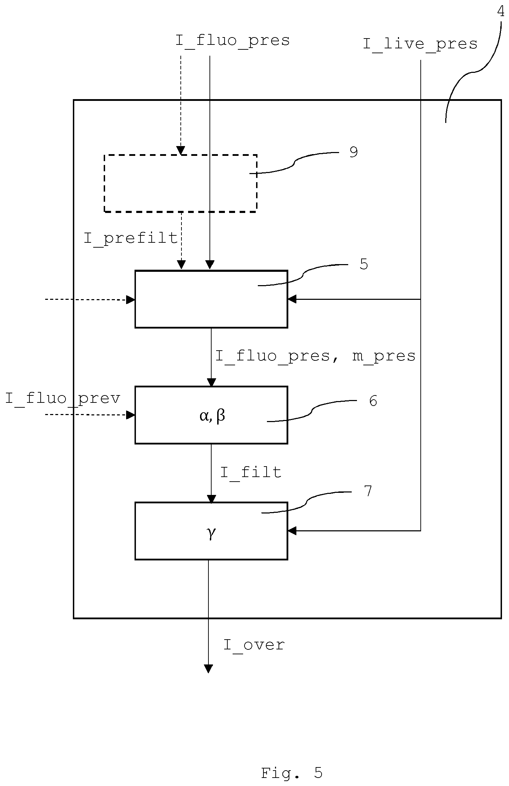

Figure 5 shows the processing unit 4 in more detail. The processing unit 4 receives a present fluorescent image I_fluo_pres and a present live image I_live_pres as input. In the motion detection module 5 of the processing unit 4, motion in the present fluorescent image I_fluo_pres and/or the present live image I_live_pres is determined. Alternatively, the present fluorescent image may be preprocessed by noise filtering in a pre-processing noise filter module 9. The pre-processing noise filter module 9, however, is optional. Furthermore, the motion detection module 5 may determine the motion in the present fluorescent image I_fluo_pres and/or the present live image I_live_pres by using signals from an external tracking system or an IMU.

The present motion m_pres and the present fluorescent image I_fluo_pres is transmitted to the adaptive motion filter module 6. In the adaptive motion filter module 6, the present fluorescent image I_fluo_pres is added to a previous filtered fluorescent image I_filt_prevby using the first factor $\alpha$ and the second factor $\beta$. The first factor $\alpha$ is proportional to the present motion m_pres. Also, the second factor $\beta$ is proportional to the first factor $\alpha$. The resulting filtered fluorescent image I_filt is transmitted to the mapping module 7, which is configured to generate overlay images by non-linearly mapping the filtered fluorescent images I_filt into the live images I_live. For the non-linear mapping, the mapping module 7 uses a non-linear amplification function $\gamma$. The processing unit 4 has the resulting overlay images I_over as an output.

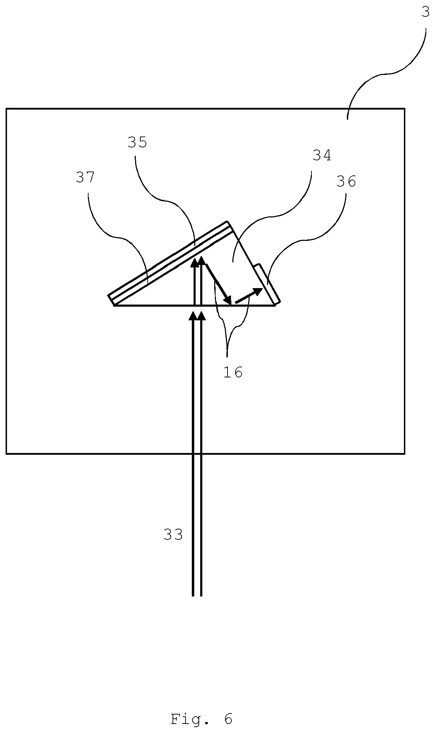

Figure 6 shows an example of the imaging unit 3 in detail. The imaging unit 3 comprises a separation means 34 which is depicted as a dichroic filter in figure 6. The light 34 which contains reflected light from the tissue 21 and also fluorescent light 16 which is emitted from the excited fluorophore, enters the dichroic filter 34 and passes through the dichroic filter 34 to the live image sensor 35. Optionally, an excitation light filter 37 can be placed between the live image sensor and the dichroic filter 34 in order to filter out excitation light 15 which is reflected from the tissue 21. In the dichroic filter 34, the fluorescent light 16 contained in the incoming light 33 is reflected on the surface of the dichroic filter two times, entering a fluorescent image sensor 36 on the right side of the dichroic filter 34.

The invention relates to a method and a device 1 for medical imaging in particular intra-operative imaging of tissue 21 of a patient's body. The device 1 comprises the illumination unit 2, the imaging unit 3, the processing unit 4 and the display unit 8. The imaging unit captures fluorescent images I_fluo and live images I_live of the same scenery. In the processing unit 4, the fluorescent images I_fluo are processed by using the detected motion between the tissue

(21) and the imaging unit (3) based on the detected motion. The filtered fluorescent images are also non-linearly mapped into the live images in order to enhance the visibility of fluorophores in the tissue resulting in an overlay image that comprises details of the live images on one hand and details of the fluorescent images on the other hand.

List of Reference Signs:

| | |
|---|---|
| 1 | device for (intra-operative) medical imaging |
| 2 | illumination unit |
| 3 | imaging unit |
| 4 | processing unit |
| 5 | motion detection module |
| 6 | adaptive motion filter module |
| 7 | mapping module |
| 8 | display unit |
| 9 | pre-processing noise filter module |
| 10 | endoscope |
| 11 | distal end of the endoscope |
| 12 | proximal end of the endoscope |
| 13 | elongated shaft of the endoscope |
| 14 | illumination light |
| 15 | excitation light |
| 16 | fluorescent light |
| 17 | fluorescent feature |
| 18 | objective of the endoscope |
| 19 | optical channel |
| 20 | vessel (organ) |
| 21 | tissue |
| 22 | border region |
| 23 | high intensity region |
| 24 | region assigned to a first color |
| 25 | region assigned to a second color |
| 26 | region assigned to a third color |
| 27 | linear function |
| 28 | square root as amplification function |
| 29 | root function of higher degrees as amplification function |
| 30 | first assigned color range |
| 31 | second assigned color range |
| 32 | third assigned color range |
| 33 | reflected light |
| 34 | separation means (dichroic filter) |
| 35 | live image sensor |
| 36 | fluorescent image sensor |
| 37 | excitation light filter |
| 38 | inertial measurement unit |
| A | illuminated area |
| I_live | live images |
| I_fluo | fluorescent images |
| I fluo_pres | present fluorescent image |
| I_filt | filtered image |
| I_filt_prev | previous filtered image |
| I_over | overlay image |
| m | motion |
| $\alpha$ | first factor |
| $\beta$ | second factor |
| $\gamma$ | amplification function |

**Claims**

1. Device (1) for medical imaging of tissue (21) of a patient's body comprising:

   an illumination unit (2) configured to illuminate an area (A) of the tissue (21) by emitting illumination light (14) containing at least excitation light (15) which excites fluorophores in the tissue (21) to emit fluorescent light (16);
   an imaging unit (3) configured to capture fluorescent images (I_fluo) and corresponding live images (I_live) of the illuminated area (A);
   a processing unit (4) configured to receive the fluorescent images (I_fluo) and the live images (I_live) from the imaging unit (3), wherein the processing unit (4) further includes:

   a motion detection module (5) configured to determine motion between the tissue (21) and the imaging unit (3);
   an adaptive motion filter module (6) configured to generate filtered fluorescent images (I_filt) by applying motion based adaptive filtering to the fluorescent images (I_fluo); and
   a mapping module (7) configured to generate overlay images (I_over) by non-linearly mapping the filtered fluorescent images (I_filt) into the live images (I_live); and

   a display unit (8) configured to receive and display the overlay images (I_over).

2. Device (1) according to claim 1, **characterized in that** the generation of the filtered fluorescent images (I_filt) in the adaptive motion filter module (6) involves weighted averaging of a present fluorescent image (I_fluo_pres) and a previous filtered image (I_filt_prev), wherein the weights in the averaging are adapted to the motion determined in the motion detection module (5).

3. Device (1) according to claim 2, **characterized in that** the weights comprising a first factor ($\alpha$) and a second factor ($\beta$), wherein the first factor ($\alpha$) determines the influence of the present fluorescent image (I_fluo_pres) in relation to the previous fluorescent image (I_filt_prev) and the second factor ($\beta$) determines the amplification of the previous filtered image (I_filt_prev).

4. Device (1) according to any of the preceding claims, **characterized in that** the adaptive motion filter module (6) is configured to successively generate a filtered fluorescent image (I_filt) for a plurality of time steps, wherein the filtered fluorescent image (I_filt) is calculated as follows for a present time step:

$$\text{I\_filt} = \alpha \cdot \text{I\_fluo\_pres} + (1 - \alpha) \cdot \beta \cdot \text{I\_filt\_prev,}$$

   wherein the filtered fluorescent image (I_filt) in the present time step becomes the previous filtered image (I_filt_prev) in the next time step.

5. Device (1) according to claim 3 or 4, **characterized in that** the adaptive motion filter module (6) is configured to adapt the first factor ($\alpha$) to the motion determined in the fluorescent images (I_fluo) and/or the live images (I_live) such that the first factor ($\alpha$) decreases when the motion decreases and the first factor ($\alpha$) increases when the motion increases.

6. Device (1) according to any of the preceding claims, **characterized in that** the second factor ($\beta$) is proportional to at least the first factor ($\alpha$), preferably the second factor ($\beta$) is also proportional to the previous filtered image (I_filt_prev).

7. Device (1) according to any of the preceding claims, **characterized in that** the second factor ($\beta$) is defined to be in a predetermined range, the predetermined range preferably being between 1.00 and 1.50, preferably between 1.00 and 1.30.

8. Device (1) according to any of the preceding claims, **characterized in that** the processing unit (4) further comprises a pre-processing noise filter module (9) configured to:

   receive the fluorescent images (I_fluo) from the imaging unit (3),
   generate pre-filtered fluorescent images (I_prefilt) by applying a noise filter to the fluorescent images (I_fluo), and

transmit the pre-filtered fluorescent images (I_prefilt) to the adaptive motion filter module (6),
wherein the adaptive motion filter module (6) is configured to apply the adaptive filtering to the pre-filtered fluorescent images (I_prefilt) for generating the filtered fluorescent images (I_filt).

9. Device (1) according to any of the preceding claims, **characterized in that** the mapping module (7) is configured to apply a non-linear amplification function ($\gamma$) on the filtered fluorescent images (I_filt) in the mapping of the filtered fluorescent images (I_filt) into the live images (I_live).

10. Device (1) according to claim 9, **characterized in that** the non-linear amplification function ($\gamma$) is underlinear homogeneous.

11. Device (1) according to claim 9 or 10, **characterized in that** the value range of the amplification function ($\gamma$) is limited to a predetermined maximal value, the maximal value preferably is below 1.00, particularly below 0.80.

12. Device (1) according to any of the preceding claims, **characterized in that** the mapping module (7) is configured to assign intensity value ranges of the mapped and filtered fluorescent images (I_filt) to different visually distinguishable colors.

13. Device (1) according to any of the preceding claims, **characterized in that** the overlay image (I_over) is calculated for every time step as follows:

$$\texttt{I\_over} = (1 - \gamma(\texttt{I\_filt})) \cdot \texttt{I\_live} + \gamma(\texttt{I\_filt}) \cdot \texttt{color(I\_filt)}.$$

14. Device (1) according to any of the preceding claims, **characterized by** further comprising an endoscope (10) with a distal end (11) and proximal end (12) between which an elongated shaft (13) extends, wherein the illumination unit (2) and/or the imaging unit (2) is positioned at the distal end (11) and/or the proximal end (12) of the endoscope (10).

15. Method for medical imaging of tissue (21) of a patient's body, particularly with a device according to any of the preceding claims, including:

illuminating an area of the tissue (21) with an illumination unit (2) by emitting illumination light (14) containing at least excitation light (15) which excites fluorophores in the tissue (21) to emit fluorescent light (16);
capturing fluorescent images (I_fluo) and corresponding live images (I_live) of the illuminated area (A) with an imaging unit (3);
processing the fluorescent images (I_fluo) and the live images (I_live) with a processing unit (4) including the following steps:

determining motion between the tissue (21) and the imaging unit (3);
generating filtered fluorescent images (I_filt) by applying motion based adaptive filtering to the fluorescence images (I_fluo); and
generating overlay images (I_over) by non-linearly mapping the filtered fluorescent images (I_filt) into the live images (I_live); and

displaying the generated overlay images (I_over).

Fig. 1

Fig. 2

EP 4 437 927 A1

Fig. 3

13

Fig. 4

I_fluo_pres                    I_live_pres

I_prefilt

I_fluo_pres, m_pres

I_fluo_prev

α, β

I_filt

γ

I_over

Fig. 5

Fig. 6

Europäisches
Patentamt

European
Patent Office

Office européen
des brevets

# EUROPEAN SEARCH REPORT

Application Number

EP 23 16 6161

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP H07 250804 A (OLYMPUS OPTICAL CO) 3 October 1995 (1995-10-03) | 1,15 | INV. A61B1/00 |
| A | * paragraphs [0019] – [0027] – paragraphs [0032] – [0041]; figures 1,3 * | 2-14 | A61B1/04 A61B1/045 A61B1/06 |
| A | US 2013/096376 A1 (TAKEI SHUNJI [JP] ET AL) 18 April 2013 (2013-04-18) * paragraphs [0047] – [0048], [0161], [0168] – [0170] * | 1-15 | |
| A | US 2012/013773 A1 (YOSHINO KOICHIRO [JP] ET AL) 19 January 2012 (2012-01-19) * paragraph [0008] * | 1-15 | |

TECHNICAL FIELDS
SEARCHED      (IPC)

A61B

The present search report has been drawn up for all claims

| Place of search | Date of completion of the search | Examiner |
|---|---|---|
| Munich | 18 August 2023 | Fischer, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 16 6161

This annex lists the patent family members relating to the patent documents cited in the above-mentioned European search report.
The members are as contained in the European Patent Office EDP file on
The European Patent Office is in no way liable for these particulars which are merely given for the purpose of information.

18-08-2023

| Patent document cited in search report | | Publication date | Patent family member(s) | | Publication date |
|---|---|---|---|---|---|
| JP H07250804 | A | 03-10-1995 | NONE | | |
| US 2013096376 | A1 | 18-04-2013 | CN | 103491847 A | 01-01-2014 |
| | | | EP | 2679137 A1 | 01-01-2014 |
| | | | JP | 5355799 B2 | 27-11-2013 |
| | | | JP | WO2012169270 A1 | 23-02-2015 |
| | | | US | 2013096376 A1 | 18-04-2013 |
| | | | WO | 2012169270 A1 | 13-12-2012 |
| US 2012013773 | A1 | 19-01-2012 | CN | 102361583 A | 22-02-2012 |
| | | | EP | 2412291 A1 | 01-02-2012 |
| | | | JP | 5242479 B2 | 24-07-2013 |
| | | | JP | 2010227253 A | 14-10-2010 |
| | | | US | 2012013773 A1 | 19-01-2012 |
| | | | WO | 2010109950 A1 | 30-09-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10694152 B2
- US 2019167083 A1
- JP 2012085917 A
- JP 2012085916 A
- JP 2003070721 A
- US 2008204600 A1

- US 2017281093 A1
- EP 3469420 A1
- US 2021052226 A1
- EP 3834407 A1
- US 5667474 A
- JP H07250804 A